# EUROPEAN PATENT SPECIFICATION

(11) **EP 1 795 131 B1**
(45) Date of publication and mention of the grant of the patent: **25.07.2012**
(21) Application number: 06025194.9
(22) Date of filing: 06.12.2006
(51) Int. Cl.: A61B 8/08, G01S 15/89, A61N 7/02

(54) **High intensity focused ultrasound system**
System für fokussierten Ultraschall hoher Intensität
Système à ultrasons focalisés à haute intensité

(30) Priority: 09.12.2005 KR 20050120581
(43) Date of publication of application: 13.06.2007
(73) Proprietor: SAMSUNG MEDISON CO., LTD., Kangwon-do 250-875 (KR)
(72) Inventor: Jeong, Mok Kun, Nowon-gu, Seoul 139-768 (KR); Yoon, Ra Young, Gangnam-gu Seoul 135-280 (KR)
(74) Representative: Schmid, Wolfgang

(56) References cited:
- WO-A-2005/107601
- US-A1- 2004 267 253
- US-B1- 6 428 477
- SAKUMA I ET AL: "Navigation of high intensity focused ultrasound applicator with an integrated three-dimensional ultrasound imaging system" MEDICAL IMAGE COMPUTING AND COMPUTER-ASSISTED INTERVENTION - MICCAI 2002. 5TH INTERNATIONAL CONFERENCE. PROCEEDINGS, PART II (LECTURE NOTES IN COMPUTER SCIENCE VOL.2489) SPRINGER-VERLAG BERLIN, GERMANY, 2002, pages 133-139, XP002425441 ISBN: 3-540-44225-1

## Description

### BACKGROUND

### 1. Field

The present invention generally relates to an ultrasound diagnostic system, and more particularly to a high intensity focused ultrasound system.

### 2. Background

An ultrasound diagnostic system has become an important and popular diagnostic tool due to its wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional (2D or 3D) diagnostic images of a target object. The ultrasound diagnostic system generally uses a probe including an array transducer having a plurality of transducer elements to transmit and receive ultrasound signals. The ultrasound diagnostic system forms ultrasound images of the internal structures of the target object by electrically exciting the transducer elements to generate ultrasound pulses that travel into the target object. The ultrasound pulses produce ultrasound echoes since they are reflected from a discontinuous surface of acoustic impedance of the internal structure, which appears as discontinuities to the propagating ultrasound pulses. Various ultrasound echoes return to the array transducer and arc converted into electrical signals, which are amplified and processed to produce ultrasound data for forming an image of the internal structure of the target object.

In particular, a high intensity focused ultrasound system is renowned to produce excellent treatment effects in the medical field. The high intensity focused ultrasound system uses high intensity focused ultrasound in order to remove a lesion (e.g., malignant tumor) from the human tissues. Conventionally, the high intensity focused ultrasound system transmits high intensity focused ultrasound onto the lesion to remove the lesion, while displaying a 2-dimensional ultrasound image of said lesion. However, the conventional high intensity focused ultrasound system is characterized by the following problems.

First, when the lesion is to be removed by using the high intensity focused ultrasound system, a region for treating the lesion is defined by the number of slices containing the lesion and distances between the slices. In treatment, the lesion is checked and examined by using a 2D ultrasound image of each slice. Since high intensity focused ultrasound used for removing the lesion is 3-dimensionally formed with a predetermined width, such an ultrasound beam may affect the tissues of slices that are adjacent to the currently treated slice during treatment. Thus, it is necessary to check other slices adjacent to the currently treated slice. Conventionally, a treatment head of the high intensity focused ultrasound system should be moved to provide ultrasound images of other slices. As such, it is impossible to check the other slices except the currently treated slice. Therefore, it is difficult to accurately check and examine the overall tissue state in real time during treatment

Further, the conventional high intensity focused ultrasound system employs a phased array probe for transmitting ultrasound signals to the lesion and receiving ultrasound echo signals reflected from the lesion. Such a probe is typically a sector probe, which provides a 2D ultrasound image at a low resolution for the lesion located deep in the body.

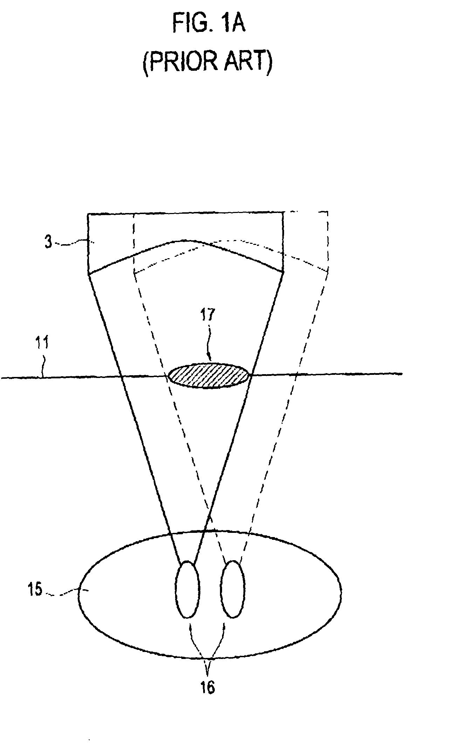

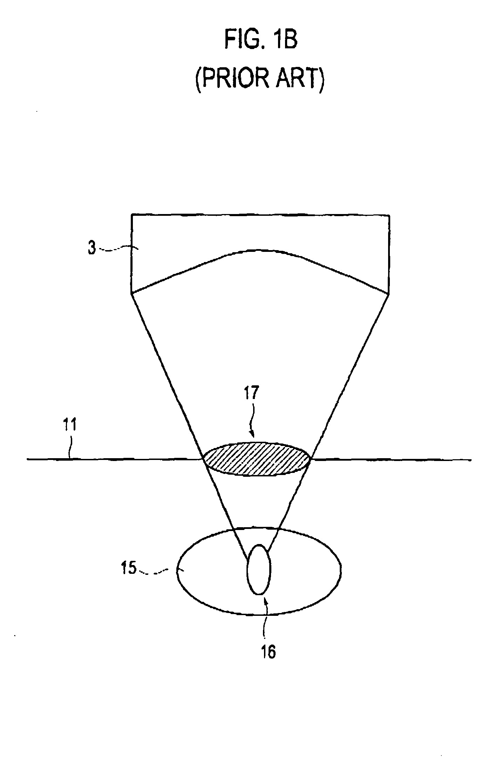

Moreover, the conventional high intensity focused ultrasound system can damage the skin. For example, when the size of a lesion 15 is significant as shown in FIG. 1A, a treatment head 3 should be moved to change a focus 16 of high intensity focused ultrasound generated from the treatment head 3. In such a case, since their sound fields overlap, damage (e.g., bum 17) to the skin 11 may be caused. Further, when the lesion 15 is disposed close to the skin 11 as shown in FIG. 1B, this may also damage the skin 11. Generally, the treatment head 3 of the conventional high intensity focused ultrasound system is installed at a bottom of the probe to be faced to the skin 11, thereby making it difficult to check the skin damage in real time.

US 2004/0267253 A1 describes a system, a method and an apparatus for visualization or imaging of the transverse carpal ligament and surrounding structures of a hand of a patient, and treatment of the transverse carpal ligament for performing non-invasive carpal tunnel release. The system utilizes ultrasound waves preferably in the high frequency range and cavitations to image the transverse carpal ligament (TCL), record its location in three-dimensional space, and perform precision treatment on the transverse carpal ligament.

In WO 2005/107601 A2 a HIFU system is disclosed which may automatically generate a proposed treatment plan for treating a tissue treatment area with HIFU therapy. In one example, the proposed treatment plan includes a plurality of treatment sites selected based on a three-dimensional model generated from ultrasound data. In another example, the proposed treatment plan excludes portions of the tissue treatment area corresponding to blood flow, such as the neuro-vascular bundles when treating the prostate.

US 6,428,477 B1 describes a fully steerable two-dimensional ultrasound array which delivers a therapy by steering and selective focusing of beams. The ultrasound array may also include an imaging functionality to simultaneously perform diagnostic imaging and delivery of a therapy. The two-dimensional ultrasound array may include a controller that controls beam forming and focusing to scan the focal point of the beam in a pattern within an identified structure of a image. Tissue is thus scanned using a sharply focused beam that is suitable for delivering a therapy such as a hyperthermia therapy or a therapy utilizing delivery of a pharmaceutical via microspheres.

Document XP-002425441 "Navigation of High Intensity Focused Ultrasound Applicator with an integrated Three-Dimensional Ultrasound Imaging System" by Ichiro Sakuma et al. describes the integration of a three-dimensional ultrasound imaging system in a HIFU applicator.

### BRIEF DESCRIPTION OF THE DRAWINGS

Arrangements and embodiments may be described in detail with reference to the following drawings in which like reference numerals refer to like elements and wherein:

FIGS. 1A and 1B are diagrams illustrating a conventional high intensity focused ultrasound system capable of causing skin damage;

FIG. 2 illustrates a high intensity focused ultrasound system constructed in accordance with one embodiment of the present invention;

FIG. 3 illustrates a combination treatment head constructed in accordance with one embodiment of the present invention;

FIG. 4 illustrates a combination treatment head constructed in accordance with another embodiment of the present invention;

FIGS. 5A to 5D illustrate various activation states for elements of a transducer included in a 2D array probe;

FIG. 6 illustrates a combination treatment head constructed in accordance with yet another embodiment of the present invention; and

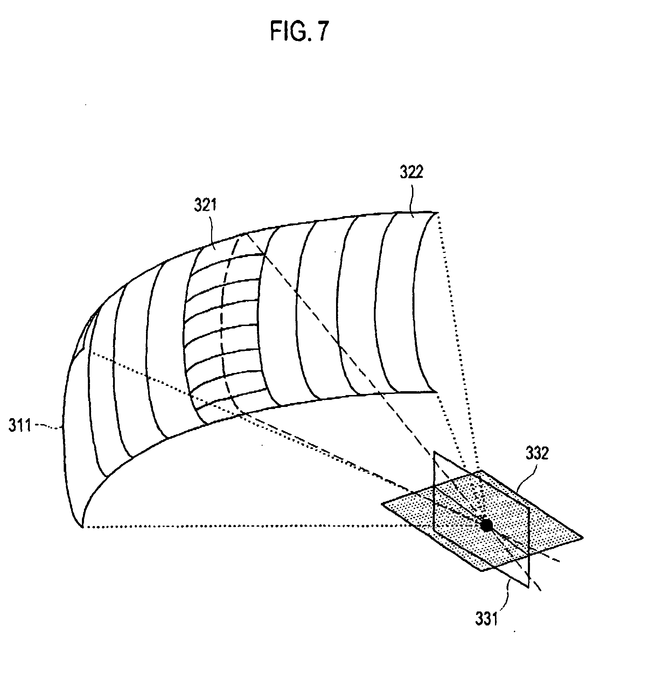

FIG. 7 illustrates an ultrasound image formed by the combination treatment head constructed in accordance with still yet another embodiment of the present invention.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

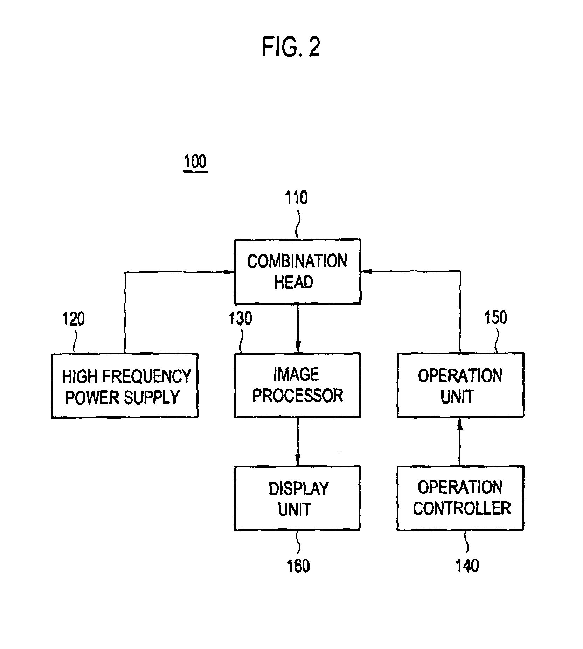

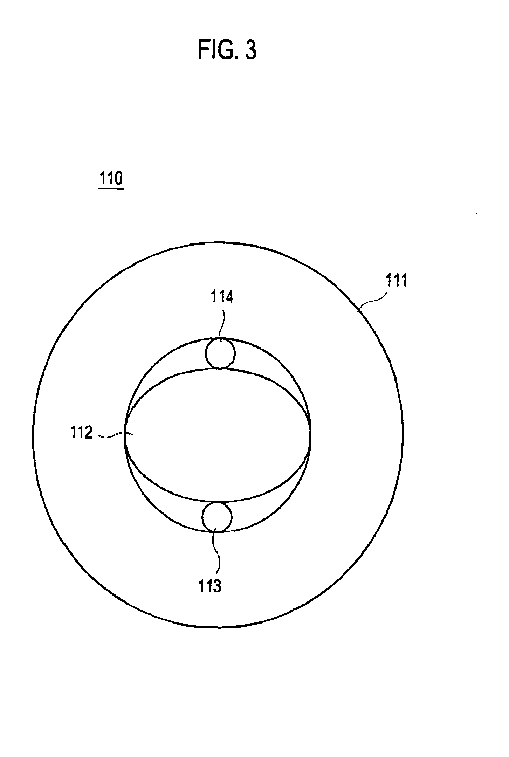

One embodiment of the present invention will be described below with reference to FIGS. 2 and 3. FIG. 2 illustrates a high intensity focused ultrasound system constructed in accordance with one embodiment of the present invention. However, other arrangements may also be used.

As shown in FIG. 2, a high intensity focused ultrasound system 100 may include a combination treatment head (combination head) 110, a high frequency power supply 120, an image processor 130, an operation controller 140, an operation unit 150 and a display unit 160. Further, the high intensity focused ultrasound system 100 may also include a container for containing a medium, which is disposed between the combination treatment head 110 and a human body (i.e., skin). Such a medium is used to transmit a high intensity focused ultrasound generated from the combination treatment head 110 to a lesion in the human body. The above container may be a water tank, a water bag and the like.

As shown in FIG. 3, the combination treatment head 110 may include a high intensity ultrasound transducer 111, a probe 112, an imaging unit 113 and a light source 114. The high intensity ultrasound transducer 111 may generate a high intensity focused ultrasound based on high frequency power provided from the high frequency power supply 120. It then transmits the high intensity focused ultrasound onto the lesion in the human body so as to remove the lesion from the human tissues. The probe 112 may transmit focused ultrasound signals to the lesion along a transmit scanline and receive ultrasound echo signals reflected from the lesion to acquire a 3D ultrasound image of the lesion in the body. The probe 112 may be configured as a 3D probe to obtain a 3D ultrasound image. The image unit 113 may produce a real-time image of the skin disposed between the combination treatment head 110 and the lesion, to which the high intensity focused ultrasound is transmitted from the combination treatment head 110. Thus, the skin can be prevented from being damaged (i.e., burned) by the high intensity focused ultrasound through using the real-time image of the skin. The imaging unit 113 may be any imaging device (e.g., miniature camera, CCTV camera, etc.) capable of being installed in the combination treatment head 110. The light source 114 may illuminate light to the skin, which allows the imaging unit 113 to produce a brighter skin image. The light source 114 may be any light source (e.g., high-brightness LED) capable of being installed in the combination treatment head 110.

Referring now back to FIG. 2, the high frequency power supply 120 may generate a high frequency power to be applied to the high intensity ultrasound transducer 111 of the combination treatment head 110. The image processor 130 may form a 3D ultrasound image of the lesion based on the ultrasound echo signals transmitted from the probe 112 and a skin image based on the skin image signals transmitted from the imaging unit 113. It may then form a 3D ultrasound image of the lesion based on the ultrasound echo signals and a skin image based on the skin image signals. Although not shown in FIG. 2, the image processor 130 may further include a beamformer, an image signal processor, a scan converter, a rendering unit and the like. The operation controller 140 may produce control signals for controlling the operation of the combination treatment head 110 such that the high intensity focused ultrasound generated from the combination treatment head 110 is transmitted to the lesion. The operation unit 150 may operate the combination treatment head 110 based on the control signals outputted from the operation controller 140. The display unit 160 may display the 3D ultrasound image of the lesion and the skin image formed in the image processor 130.

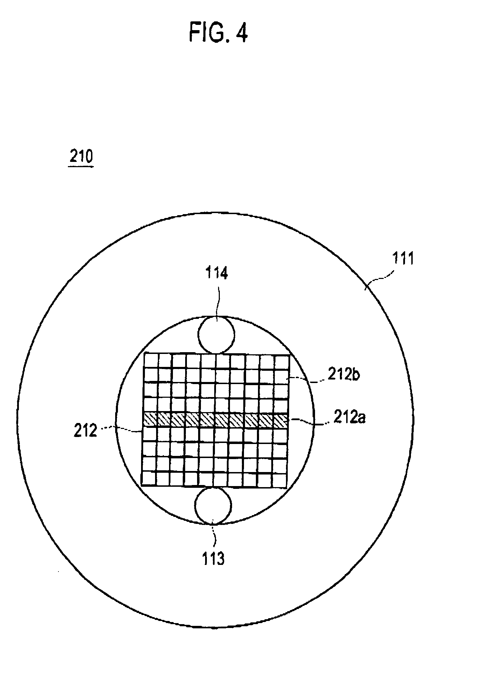

Another embodiment of the present invention will be described below with reference to FTGS. 4 to 5D. FIG. 4 illustrates a combination treatment head constructed in accordance with another embodiment of the present invention.

As shown in FIG. 4, a combination treatment head 210 may include a high intensity ultrasound transducer 111, a 2D array probe 212, an imaging unit 113 and a light source 114. In the combination treatment head 210, the high intensity ultrasound transducer 111, the imaging unit 113 and the light source 114 may have substantially the same functions as those in the combination treatment head 110 shown in FIG. 3. The same reference numerals are given to avoid any repeated descriptions thereof.

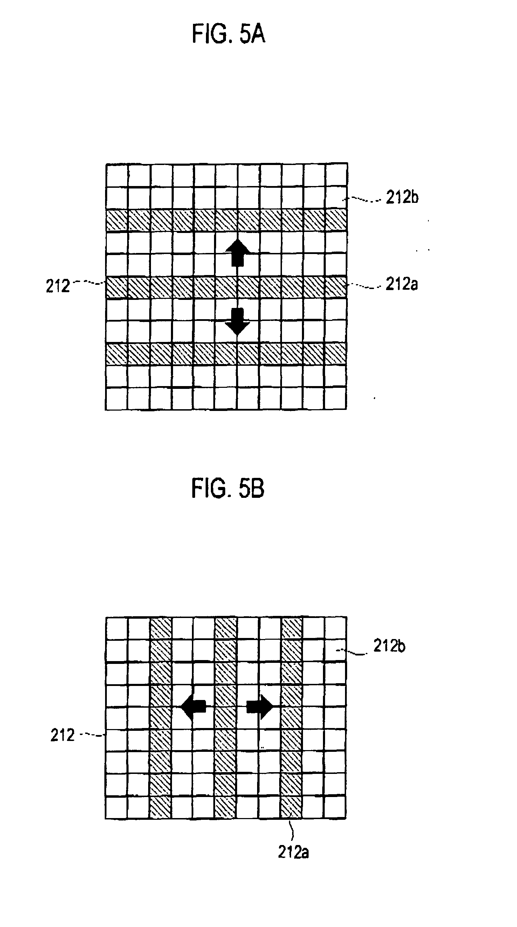

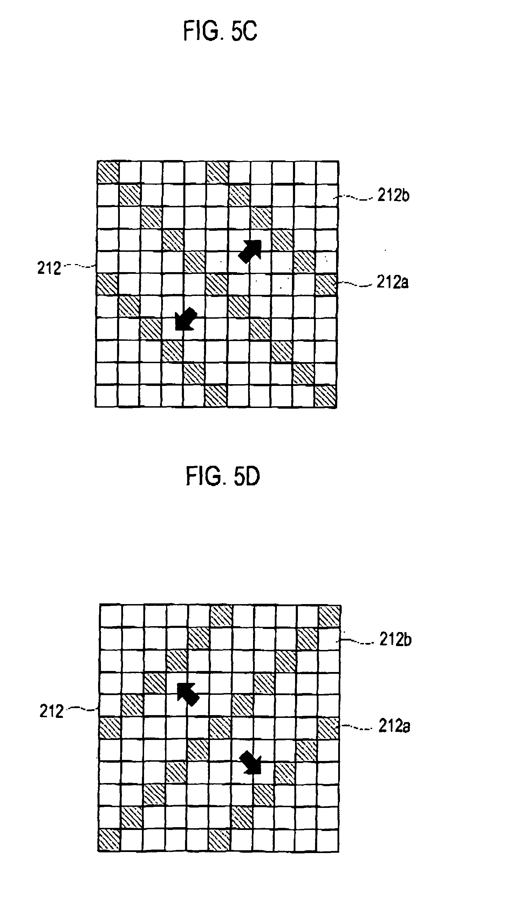

The 2D array probe 212 may include a transducer having M x N elements. The 2D array probe 212 may acquire ultrasound echo signals for forming a 3D ultrasound image while altering the activation of the elements of the transducer. As shown in FIG. 4, the elements may be divided into two groups of activated elements 212a and inactivated elements 212b. Moreover, the elements of the 2D array probe 212 may have various activation states such as horizontal activation (FIG. 5A), vertical activation (FIG. 5B) and diagonal activation (FIGS. 5C and 5D).

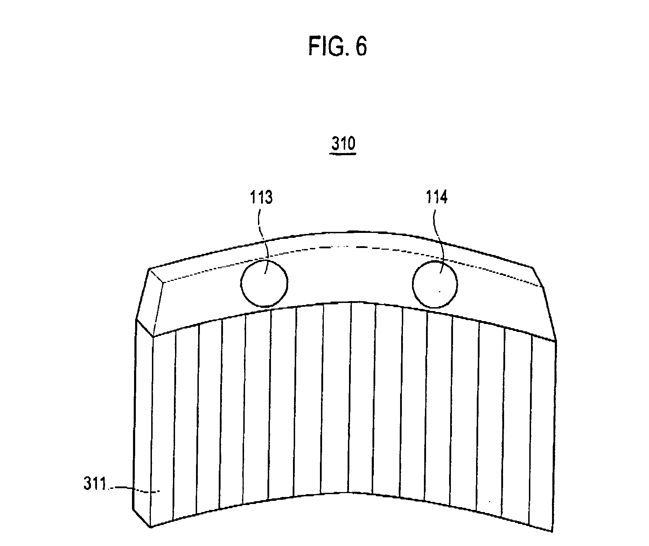

A further embodiment of the present invention will be described below with reference to FIG. 6.

FIG. 6 illustrates a combination treatment head constructed in accordance with yet another embodiment of the present invention. As shown in FIG. 6, a combination treatment head 310 may include a transducer 311, an imaging unit 113 and a light source 114. In the combination treatment head 310, the imaging unit 113 and the light source 114 may have substantially the same functions as those in the combination treatment bead 110 shown in FIG. 3. The same reference numerals are given to avoid any repeated descriptions thereof.

The transducer 311 may produce high intensity focused ultrasound for removing the lesion from the tissues in the body and ultrasound signals for forming a 3D ultrasound image of the lesion. Tt may alternately transmit the high intensity focused ultrasound for removing the lesion and the ultrasound signals for forming the 3D ultrasound image onto the lesion in the body. The high intensity focused ultrasound for removing the lesion and the ultrasound signals for forming the 3D ultrasound image produced by the transducer 311 may have different frequencies, powers and transmit waveforms. In such a case, the transducer 311 may be configured with elements arranged in a concave array such that the focus of the high intensity focused ultrasound is positioned at an image plane acquired by the ultrasound signals for forming the 3D ultrasound image of the lesion.

Further, as shown in FTG. 7, the transducer 311 may form ultrasound images 331 and 332 perpendicular to each other by arranging the central elements 321 to be smaller than the other elements 322 in a different way. By using the ultrasound image 331 perpendicular to the ultrasound image 332, the shape and size of the lesion can be more accurately examined.

As described above, in the present disclosure, the 3D probe or the 2D array probe is used instead of the phased array probe, thereby making it possible to check the slices adjacent to the present slice as well as the present slice by using a motor in the 3D probe or altering the activation of the transducer elements included in the 2D array probe without moving the combination treatment head of the high intensity focused ultrasound system. Thus, the treatment state can be checked and examined in real time. Further, the ultrasound image of the lesion at a high resolution can be obtained in case of using the 2D array probe.

Further, in the present disclosure, the imaging unit and the light source are disposed close to the probe of the combination treatment head to provide a real-time image of the skin existing between the combination treatment head and the lesion. Thus, the skin state can be checked and examined in real time to prevent the skin from being damaged.

An embodiment may be achieved in whole or in part by a high intensity focused ultrasound system, which includes a combination head unit for: producing first ultrasound signals for removing a first region to focus and transmit the first ultrasound signals onto the first region; producing second ultrasound signals for forming a 3D ultrasound image of the first region to focus and transmit the second ultrasound signals onto the first region; receiving ultrasound echo signals reflected from the first region; and acquiring image signals for forming an image of a second region, wherein the first and second ultrasound signals penetrate through the second region onto the first region. It also includes: a high frequency power supply unit for generating a high frequency power to be applied to the combination head unit; an image processor for forming the 3D ultrasound image of the first region based on the ultrasound echo signals and the image of the second region based on the image signals; an operation controller for producing control signals for controlling an operation of the combination head unit such that at the first ultrasound signals are focused on the first region; an operating unit for operating the combination head unit based on the control signals outputted from the operation controller, and a display unit for displaying the 3D ultrasound image of the first region and the image of the second region.

Any reference in this specification to "one embodiment," "an embodiment," "example embodiment," etc. means that a particular feature, structure or characteristic described in connection with the embodiment is included in at least one embodiment of the invention. The appearances of such phrases in various places in the specification are not necessarily all referring to the same embodiment. Further, when a particular feature, structure of characteristic is described in connection with any embodiment, it falls within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art

## Claims

1. A high intensity focused ultrasound system (100), comprising:
a combination head unit (110, 310) for producing first ultrasound signals for removing a first region to focus and transmit the first ultrasound signals onto the first region, the combination head unit being configured to produce second ultrasound signals for forming a 3D ultrasound image of the first region to focus and transmit the second ultrasound signals onto the first region, the combination head unit further being configured to receive ultrasound echo signals reflected from the first region;
a high frequency power supply unit (120) for generating a high frequency power to be applied to the combination head unit (110);
an image processor (130) for forming the 3D ultrasound image of the first region based on the ultrasound echo signals; an operation controller (140) for producing control signals for controlling an operation of the combination head unit (110) such that the first ultrasound signals are focused on the first region;
an operating unit (150) for operating the combination head unit based on the control signals outputted from the operation controller (140); and
a display unit (160) for displaying the 3D ultrasound image of the first region,
**characterized in that** the combination head unit (110) includes an imaging unit (113) including one of a miniature camera and a CCTV camera for acquiring image signals for forming an image of a second region, wherein the first and second ultrasound signals penetrate through the second region onto the first region, wherein the image processor (130) forms the image of the second region based on the image signals and wherein the display unit (160) further displays the image of the second region.

2. The high intensity focused ultrasound system of Claim 1, wherein the combination head unit (110) includes:
a first transducer (111) for producing the first ultrasound signals based on the high frequency power applied from the high frequency power supply unit to focus and transmit the first ultrasound signals onto the first region; and
a probe (112) for producing the second ultrasound signals to focus and transmit the second ultrasound signals onto the first region, the probe further being configured to receive ultrasound echo signals reflected from the first region.

3. The high intensity focused ultrasound system of Claim 2, wherein the probe (112) is a 3D probe.

4. The high intensity focused ultrasound system of Claim 2, wherein the probe (112) is a 2D array probe including a transducer with a number of elements.

5. The high intensity focused ultrasound system of Claim 1, wherein the combination head unit (110) further includes a light source for illuminating light to the second region imaged by the imaging unit.

6. The high intensity focused ultrasound system of Claim 5, wherein the light source (114) is a high-brightness LED.

7. The high intensity focused ultrasound system of Claim 1, wherein the combination head unit (310) includes:
a second transducer (311) for producing the first and second ultrasound signals to alternately focus and transmit the first and second ultrasound signals onto the first region.

8. The high intensity focused ultrasound system of Claim 7, wherein the second transducer (311) has a number of elements arranged in a concave array.

9. The high intensity focused ultrasound system of Claim 7, wherein the combination head unit (310) includes a light source (114) for illuminating light to the second region imaged by the imaging unit.

10. The high intensity focused ultrasound system of Claim 9, wherein the light source (114) is a high-brightness LED.

## Patentansprüche

1. System (100) für fokussierten Ultraschall hoher Intensität, welches Folgendes aufweist:
eine kombinierte Kopfeinheit (110,310) zum Erzeugen erster Ultraschallsignale zum Entfernen eines ersten Bereichs, um die ersten Ultraschallsignale zu fokussieren und auf den ersten Bereich zu übertragen, wobei die kombinierte Kopfeinheit dafür vorgesehen ist, zweite Ultraschallsignale zum Bilden eines 3D-Ultraschallbilds des ersten Bereichs zu produzieren, um die zweiten Ultraschallsignale zu fokussieren und auf den ersten Bereich zu übertragen, wobei die kombinierte Kopfeinheit des Weiteren dafür vorgesehen ist, von dem ersten Bereich reflektierte Ultraschallechosignale zu empfangen;
eine Hochfrequenz-Energieversorgungseinheit (120) zum Erzeugen eines Hochfrequenzstroms, der der kombinierten Kopfeinheit (110) zugeführt werden soll;
einen Bildprozessor (130) zum Bilden des 3D-Ultraschallbilds des ersten Bereichs basierend auf den Ultraschallechosignalen; ein Betriebssteuergerät (140) zum Produzieren von Steuersignalen zum Steuern eines Betriebs der kombinierten Kopfeinheit (110) derart, dass die ersten Ultraschallsignale auf den ersten Bereich fokussiert werden;
eine Bedieneinheit (150) zum Bedienen der kombinierten Kopfeinheit basierend auf den von dem Betriebssteuergerät (140) ausgegebenen Steuersignalen; und eine Anzeigeeinheit (160) zum Anzeigen des 3D-Ultraschallbilds des ersten Bereichs,
**dadurch gekenntzeichnet,** dass
die kombinierte Kopfeinheit (110) eine Bildeinheit (113) mit einer Kleinbildkamera oder einer CCTV-Kamera zum Erlangen von Bildsignalen zum Bilden eines zweiten Bereichs aufweist, wobei die ersten und zweiten Ultraschallsignale durch den zweiten Bereich auf den ersten Bereich durchdringen, wobei der Bildprozessor (130) das Bild des zweiten Bereichs basierend auf den Bildsignalen bildet, und wobei die Anzeigeeinheit (160) des Weiteren das Bild des zweiten Bereichs anzeigt.

2. System für fokussierten Ultraschall hoher Intensität nach Anspruch 1, wobei die kombinierte Kopfeinheit (110) Folgendes aufweist:
einen ersten Transducer (111) zum Erzeugen der ersten Ultraschallsignale basierend auf dem von der Hochfrequenz-Energieversorgungseinheit zugeführten Hochfrequenzstrom, um die ersten Ultraschallsignale zu fokussieren und auf den ersten Bereich zu übertragen; und
eine Sonde (112) zum Erzeugen der zweiten Ultraschallsignale, um die zweiten Ultraschallsignale zu fokussieren und auf den ersten Bereich zu übertragen, wobei die Sonde des Weiteren dafür vorgesehen ist, von dem ersten Bereich reflektierte Ultraschallsignale zu empfangen.

3. System für fokussierten Ultraschall hoher Intensität nach Anspruch 2, wobei die Sonde (112) eine 3D-Sonde ist.

4. System für fokussierten Ultraschall hoher Intensität nach Anspruch 2, wobei die Sonde (112) eine 2D-Array-Sonde mit einem Transducer mit einer Vielzahl von Elementen ist.

5. System für fokussierten Ultraschall hoher Intensität nach Anspruch 1, wobei die kombinierte Kopfeinheit (110) des Weiteren eine Lichtquelle zum Ausstrahlen von Licht zu dem durch die Bildeinheit abgebildeten zweiten Bereich aufweist.

6. System für fokussierten Ultraschall hoher Intensität nach Anspruch 5, wobei die Lichtquelle (114) eine LED mit hoher Helligkeit ist.

7. System für fokussierten Ultraschall hoher Intensität nach Anspruch 1, wobei die kombinierte Kopfeinheit (310) Folgendes aufweist:
einen zweiten Transducer (311) zum Erzeugen der ersten und zweiten Ultraschallsignale, um abwechselnd die ersten und zweiten Ultraschallsignale zu fokussieren und auf den ersten Bereich zu übertragen,

8. System für fokussierten Ultraschall hoher Intensität nach Anspruch 7, wobei der zweite Transducer (311) eine Vielzahl von Elementen aufweist, die in einer konkaven Reihe angeordnet sind.

9. System für fokussierten Ultraschall hoher Intensität nach Anspruch 7, wobei die kombinierte Kopfeinheit (310) eine Lichtquelle (114) zum Ausstrahlen von Licht zu dem durch die Bildeinheit abgebildeten zweiten Bereich aufweist.

10. System für fokussierten Ultraschall hoher Intensität nach Anspruch 9, wobei die Lichtquelle (114) eine LED mit hoher Helligkeit ist.

## Revendications

1. Dispositif de focalisation d'ultrasons de forte intensité (100) comportant une unité à tête combinée (110, 310) pour produire des premiers signaux ultrasoniques pour retirer une première zone à focaliser et transmettre les premiers signaux ultrasoniques sur cette zone, ladite unité à tête combinée étant agencée pour produire des seconds signaux ultrasoniques pour former une image ultrasoniques en 3D de la première zone à focaliser et pour transmettre les seconds signaux ultrasoniques sur le première zone, l'unité à tête combinée étant en outre agencée pour recevoir les signaux de l'écho ultrasonique réfléchi par la première zone ;
une unité d'alimentation en énergie haute fréquence (120) pour générer de la puissance électrique haute fréquence à appliquer à l'unité à tête combinée (110) ;
un dispositif de traitement d'image (130) pour former l'image ultrasonique 3D sur la première zone basée sur les signaux de l'écho ultrasonique;
un dispositif de contrôle opératoire (140) pour produire des signaux de contrôle pour vérifier le fonctionnement de l'unité à tête combinée (110) comme vérifier que les premiers signaux ultrasoniques sont focalisés sur la première zone ;
une unité opératoire (150) pour actionner l'unité à tête combinée sur la base des signaux de contrôle extraits du dispositif de contrôle opératoire (140) ; et
une unité d'affichage (160) pour afficher l'image ultrasonique en 3D sur la première zone,
**caractérisé en ce que** l'unité à tête combinée (110) comporte une unité de création d'image (113) comprenant soit une caméra miniature soit une caméra CCTV pour acquérir les signaux d'image pour former une image sur la deuxième zone, les premiers et les seconds signaux ultrasoniques pénétrant à travers la seconde zone sur la première zone, le dispositif de traitement d'image (130) formant l'image sur la seconde zone sur la base des signaux d'image et l'unité d'affichage (160) affichant également l'image sur la seconde zone.

2. Dispositif de focalisation d'ultrasons de forte intensité selon la revendication 1, dans lequel l'unité à tête combinée (110) comprend :
un premier transducteur (111) pour produire des premiers signaux ultrasoniques sur base de la puissance haute fréquence appliquée par l'unité d'alimentation en énergie électrique à haute fréquence pour focaliser et transmettre les signaux ultrasoniques sur la première zone, et
un premier échantillonnage (112) pour produire les signaux ultrasoniques pour focaliser et transmettre les seconds signaux ultrasoniques sur la première zone, l'échantillonnage étant en outre agencé pour recevoir les signaux ultrasoniques de l'écho réfléchis par la première zone.

3. Dispositif de focalisation d'ultrasons de forte intensité selon la revendication 2, dans lequel l'échantillonnage (112) est en 3D.

4. Dispositif de focalisation d'ultrasons de forte intensité selon la revendication 2, dans lequel l'échantillonnage (112) est un réseau en 2D qui comprend un transducteur avec une quantité d'éléments.

5. Dispositif de focalisation d'ultrasons de forte intensité selon la revendication 1, dans lequel l'unité à tête combinée (110) comporte en outre une source de lumière pour éclairer la seconde zone représentée par l'unité d'affichage.

6. Dispositif de focalisation d'ultrasons de forte intensité selon la revendication 5, dans lequel la source de lumière (114) est une LED de luminosité intense.

7. Dispositif de focalisation d'ultrasons de forte intensité selon la revendication 1, dans lequel l'unité à tête combinée (310) comporte :
un second transducteur (311) pour produire les premiers et les seconds signaux ultrasoniques pour focaliser alternativement et transmettre les premiers et les seconds signaux ultrasoniques sur la première zone.

8. Dispositif de focalisation d'ultrasons de forte intensité selon la revendication 7, dans lequel le second transducteur (311) comporte de nombreux éléments disposés selon un réseau concave.

9. Dispositif de focalisation d'ultrasons de forte intensité selon la revendication 7, dans lequel l'unité à tête combinée (310) comporte une source lumineuse (114) pour éclairer la seconde zone représentée par l'unité d'affichage.

10. Dispositif de focalisation d'ultrasons de forte intensité selon la revendication 9, dans lequel la source (114) est une LED de luminosité intense.
